# FASCICULE DE BREVET EUROPEEN

(11) **EP 4 048 764 B1**
(45) Date de publication et mention de la délivrance du brevet: **17.07.2024**
(21) Numéro de dépôt: 20842050.5
(22) Date de dépôt: 27.11.2020
(51) Int. Cl.: C11B 1/00, C11B 1/06, C11B 3/00

(54) **PROCÉDÉ D' EXTRACTION CONTINUE DE L'HUILE D'ARGAN EXTRA-VIERGE A USAGES MULTIPLES**
VERFAHREN ZUR KONTINUIERLICHEN EXTRAKTION VON NATURREINEM ARGANÖL EXTRA FÜR VERSCHIEDENE ANWENDUNGEN
METHOD FOR CONTINUOUS EXTRACTION OF EXTRA-VIRGIN ARGAN OIL FOR MULTIPLE USES

(30) Priorité: 21.10.2019 MA 47244
(43) Date de publication de la demande: 31.08.2022
(73) Titulaire: Iris Cosmetologie, MARRAKECH, 40110 (MA)
(72) Inventeur: BITAR, Khalid, MARRAKECH, 40110 (MA)
(74) Mandataire: Patent 42
(86) Numéro de dépôt international: PCT/MA2020/000015
(87) Numéro de publication internationale: WO 2021/080405

(56) Documents cités:
- CN-A- 107 650 414
- YU LINA ET AL: "Products and Properties of Components from Heat-Denatured Peanut Meal Following Solid-State Fermentation by Aspergillus oryzae and Saccharomyces cerevisiae", FERMENTATION, vol. 9, no. 5, 28 April 2023 (2023-04-28), pages 425, XP093138230, ISSN: 2311-5637, DOI: 10.3390/fermentation9050425
- BADR EDDINE ET AL: "Effect of filtration on virgin argan oil: Quality and Stability", ENVIRON. SCI, 1 January 2015 (2015-01-01), pages 2871 - 2877, XP055781326, Retrieved from the Internet <URL:https://www.jmaterenvironsci.com/Document/vol6/vol6_N10/338-JMES-1557-2015-Kartah.pdf> [retrieved on 20210302]
- CHARROUF ZOUBIDA ET AL: "Should the Amazigh Diet (Regular and Moderate Argan-Oil Consumption) have a Beneficial Impact on Human Health?", CRITICAL REVIEWS IN FOOD SCIENCE AND NUTRITION, vol. 50, no. 5, 5 April 2010 (2010-04-05), USA, pages 473 - 477, XP055781325, ISSN: 1040-8398, DOI: 10.1080/10408390802544520
- HILALI MILOUDI ET AL: "Influence of Origin and Extraction Method on Argan Oil Physico-Chemical Characteristics and Composition", JOURNAL OF AGRICULTURAL AND FOOD CHEMISTRY, vol. 53, no. 6, 1 March 2005 (2005-03-01), US, pages 2081 - 2087, XP055781298, ISSN: 0021-8561, DOI: 10.1021/jf040290t

## Description

### Domaine technique

L'huile d'argan marocain cultivé dans les régions d' Essaouira et Agadir est unique au monde donc sa qualité totale (nutritionnelle, bio thérapeutique, nutraceutique, cosmétique, alimentaire) doit être conservée scrupuleusement et égale. Le procédé proposé d'extraction est non destructif à froid et sans apport ni d'eau ni d'additifs. L'invention réside essentiellement sur le procédé innovant d'extraction purement physique et à froid, ainsi que son installation de production dans l'industrie alimentaire et cosmétique.

Le domaine technique de cette invention concerne les procédés et les installations de production de l'huile: d'argan; olive, noix, avocat, d'abricot; d'amande, de datte,....

La destination et les applications de ces huiles sont diversifiées: des huiles alimentaires, cosmétiques et de médication naturelle.

L'invention permet d'obtenir une huile d'argan extra vierge de qualité et sans destruction et transformation chimique.

L'avantage de la présente invention est que les huiles obtenues conservent toutes leurs caractéristiques organoleptiques: couleur, odeur, goût..., et que l'installation fonctionne en continue de manière non énergivore et selon un procédé optimisé.

### Etat de la technique

Les installations et les procédés d'extraction de l'huile d'argan sont traditionnelles au Maroc, pays de l'huile d'argan. A l'echelle mondiale, les innovations dans ce sens sont très rares. Les documents brevet ci dessous divulguent des procédés et installations d'extraction d'huile (non d'argan) avec un procédé et installation différente de la présente invention.

Le document WO2012011883A1 divulgue un procédé continu de production d'huile d'olive en chaine continue avec une presse à vis continu et un presage à chaud et à froid.

Le document CN109468167A divulgue un procédé d'extration d'huile comprenant une machine de criblage primaire, un vanneur, une décortiqueuse, une machine à dépecer, une machine de criblage calibrée, un pulvérisateur et un presseur disposés en séquence, et un réservoir de blanchiment, une plaque-et- cadre filtre-presse, filtre fin, un réservoir de désodorisation et une machine de remplissage disposés en sequence. Badr Eddine et al: "Effect of filtration on Virgin argan oil: Quality and Stability", Environ. Soi, 1 janvier 2015 (2015-01-01), p. 2871 -2877 divulgue un procédé d'extraction d'huile d'argan extra vierge. Le procédé consiste à presser et filtrer une noix d'argan grillée.

### Description technique

L'invention est définie par les revendications et concerne un processus continu de la transformation de la matière première, fruit d'argan, qui passe par plusieurs étapes jusqu'au produit fini l'huile d'argan extra vierge. Le procédé est articulé autour de deux parties: production d'amandes ou d'amandons et la presse mécanique contrôlée. Chaque partie est modulée en plusieurs modules: tamisage, calibrage, dépulpage, concassage, presse, filtration, stockage.

Le processus total est continu et articulé en trois étapes : A, B et C :
Etape 1 (A) : préparation des amandes
Etape 2 (B) : obtention de la poudre des amandes
Etape 3 (C): obtention de l'huile

Chaque étape est articulée autour de plusieurs modules:
L'étape A est composée de:
   1. : Pesage de la matière première et Tamisage A1
   2. : Calibrage: A2
   3. : Dépulpage: A3
   4. : Tri/ contrôle: A4
L'étape B:
   1. Concassage (B1)
   2. Tamisage (B2)
   3. Dépoussiérage(B3)
L'étape C:
   1. Mélangeur (C1)
   2. Alimentation de la presse (C2)
   3. Presse (C3)
   4. Décantation de l'huile (C4)
   5. Pompage de l'huile (CS)
   6. Filtration 1 (C6)
   7. Filtration 2 (C7)
   8. Pompage et stockage de l'huile (C8)

A: (Réception/Tamisage): La matière première (1) est usinée tout fraichement sans aucun traitement ni déshydratation. Les conditions du processus sont variables et tributaires du taux d'humidité qui varie de 8 à 15%. Donc la vitesse, la puissance des moteurs, la fréquence, le débit, le voltage et la consommation en énergie sont réajustés en fonction de la qualité des fruits. Les fruits sont pesés afin de contrôler le taux d'extraction et des pertes. Les fruits sont triés par un contrôle visuel et manuel puis par un tamiseur (2) pour se débarrasser des poussières et des corps étrangers.
B : (poste de calibrage) : Après le tri et le tamisage, le fruit est entraîné pour calibrage par une vis sans fin orientée par rapport au sol d'un angle de 45°. Les fruits sont placés dans une trémie (3) puis canalisés (5) grâce à un moteur de 380 v et avec une puissance de 3 KW (4) pour être calibrés (6) poussés avec une puissance de 2,2 KW. Quatre calibres sont obtenus selon la taille et la grosseur du fruit pour être évacués séparément à la machine de dépulpage.
C : (Poste de dépulpage) : Le fruit est conduit par un moteur d'une puissance de 1,5 KW vers le dépulpage (9). Le dépulpage (enlèvement de la pulpe ou de la coque). Un système basé sur des forces centrifuges appliquées par des palettes qui tournent horizontalement à grande vitesse avec une puissance relativement faible de 0,75 KW. Le fruit et selon ses dimensions et son calibre est éjecté ou propulsé par une vitesse et une force proportionnelle relative sur la paroi métallique qui entraine la rupture de la coque. La coque est aspirée par une pompe de 1,5 KW vers le stockage (10). L'amande est entrainée par pesanteur verticalement (11) puis subit une double vibration par deux moteurs de puissance 5,5 KW.
D: (Contrôle de la qualité du dépulpage): L'amande est entrainée sur un tapis (12) de contrôle visuel et manuel par un moteur de puissance de 0,75. Les amandes sont stockées (13).
   La surchauffe est absente et contrôlée. Donc l'amande sort tempérée et non chaude.
E : (Concassage) : L'étape la plus importante est le concassage. Cette étape de concassage où l'amande subit un matraquage par un système de coups par marteaux (14) et poussée par un moteur 0,55 KW (15) et un moteur moulin de 18,5 KW.
F: (tamisage): Le broyat est tamisé en 3 granulats par 2 tamiseurs (16). La plus fine est celle qui est la plus riche en huile (21), elle est véhiculée par un moteur de 0,55 KW de puissance. La fraction moyenne est récupérée pour être mélangée avec la fraction la plus fine (21). La fraction grande est entrainée pour être séparée en deux autres fractions (17). La séparation est effectuée avec un système d'éjection avec une puissance de 0,55 KW (18).
G : (raffinage ou dépoussiérage) : Au niveau de ce poste, on récupère les petites fractions légères d'une part et les sous-produits ou déchets d'autre part. La fraction fine appelée poussières est très riche en huile (19). L'autre fraction correspond à des particules de grosses tailles pauvres en huile, correspond à un sous-produit qui est la coque de l'amande ou pelure. Les fractions riches en huile sont transférées portées à la salle de presse.
H : (mélangeur) : les fractions (19) et (21) sont mélangées dans un malaxeur (21) d'une capacité d'environ 455 Kg avec une puissance de 5,55 KW. Il est lié à la presse pour l'alimentation par un système pneumatique de canalisation. Il est équipé d'un frein afin de ne pas créer une désynchronisation avec la presse.
I : (Alimentation de la presse) : la presse est articulée avec le malaxeur de façon automatique. Elle est autorégulée pour un débit d'approvisionnement précis. L'alimentation en matière première est sous contrôle: quantité, humidité et granulométrie. Elle donne le signal au malaxeur pour s'approvisionner en broyat. Une vis sans fin (22) est faite sur mesure pour ce procédé. Elle tourne à une fréquence contrôlée en générale elle est en moyenne de 7,5 tours/minute et avec une puissance de 65 KW. Des sous-produits, les tourteaux, qui sont éjectés horizontalement puis propulsés d'un bac vers un conteneur de stockage (23).
   i1 : (la presse) : Le système le plus important du procédé. Il est sous contrôle physique. La température est réglée et maîtrisée, le froid et le chaud selon les besoins soit cosmétique soit alimentaire, et ce par un système à 4 plateaux montés verticalement. Le contrôle des moteurs des pompes, la puissance, la fréquence et l'énergie.
   i2 : Vis sans fin de la presse : La vis est en acier galvanisé et email avec un alliage spécial.
J: pré-filtrations: L'huile est récupérée verticalement en dessous des fentes de la vis horizontale filtrée à travers un filtre en inox. Elle est ensuite décantée. Le surnagent est pompé par deux pompes à 1 KW et de 3 KW à travers des canalisations en inox vers la filtration (24). Le dépôt solide est un sous-produit qui est valorisé en cosmétique.
K : pompage de l'huile (24): L'huile est pompée et canalisée avec une puissance relativement basse à 0,3 KW vers la zone de filtration.
L: Filtration1 : L'huile subit deux séries de filtration chaud à 40°C et à froid avec une puissance de moteur de 1,5 Kw. La première filtration à travers des filtres passoires en inox.
M : Filtration2 : La seconde à travers des filtres en bio-cellulose (25). Un autre sous-produit résidu solide est récupéré pour la valorisation en cosmétique (27).
N: (Pompage et stockage): L'huile est récupérée par pompage avec une puissance de 1 KW dans des futs b(26).

### Aspects techniques

Description des composantes techniques du matériel de chaque poste

| A: POSTE DE RECEPTION/ TAMISAGE | |
|---|---|
| 1 | Moteur électrique |
| 2 | Variateur |
| 3 | Réducteur |
| 4 | Support antérieur |
| 5 | Support postérieur |
| 6 | Pignon de commande |
| 7 | Chaine métallique |
| 8 | Pignon de renvoie |

| B: POSTE DE CALIBRAGE | |
|---|---|
| 1 | moteur électrique |
| 2 | Réducteur |
| 3 | Moue de la tasse |
| 4 | Seeger pour extérieur |
| 5 | Poste de régulation extérieur |
| 6 | Trémie de chargement |
| 7 | Tamis 15 |
| 8 | Tamis 15,5 |
| 8A | Tamis 17 |
| 9 | Tamis 17,5 |
| 9A | |
| 10 | Tamis 18,5 |
| 11 | Tamis 19 |
| 11A | Coussinet-bagues |
| 12 | Vérins |
| 13 | Support de transmission |
| 14 | Trémie de chargement 1 |
| 15 | Trémie de chargement 2 |
| 16 | Trémie de chargement 3 |
| 17 | Trémie de chargement 4 |
| 18 | Epine élastique |
| 19 | Support de chargement |
| 20 | Fenêtre |
| 21 | Rouleau sphérique |
| 22 | Rouleau de nettoyage |

| C : DEPULPAGE | |
|---|---|
| 1 | moteur électrique KW 7,5 4P B3 400V 50 Hz |
| 2 | Joint d'huile de poussée à billes |
| 3 | Rouleau sphérique |
| 4 | Poulie d'entrainement |
| 5 | Appui de pallier |
| 6 | Bague et rosette |
| 7 | Ceinture: croix SPA 1800 |
| 8 | Poulie de transmission |
| 9 | Pieds anti vibration |
| 10 | Indicateur de niveau |
| 11 | Joints de trémie |
| 12 | Roue à palettes double |
| 13 | Bague anti usure à polyethylene |
| 14 | Joint de cyclone |
| 15 | Feuille résistance ou anti usure du cyclone |
| 16 | Convoyeur à vague rotative |
| 17 | Joint à vague rotative |
| 18 | Vague rotative |
| 19 | Trémie double version |
| 20 | Petit volant du réglage de la trémie |

| D : Contrôle de la qualité du DEPULPAGE | |
|---|---|
| 1 | Tapis roulant entraîné par un moteur d'une puissance 0,75 KW |
| 2 | 10 personnes contrôlent visuellement la qualité de dépulpage |
| 3 | Paniers de recuperation |

| E:CONCASSAGE | |
|---|---|
| 1 | Moteur électrique/ KW 1,5 4P B3 400V - 40Hz |
| 2 | Couple de transmission |
| 3 | bague FRB |
| 4 | Support |
| 5 | Marteau rotatif |
| 6 | Espace des marteaux |
| 7 | Ronde rotation marteaux |
| 8 | Bagues à billes |
| 9 | Vis |
| 10 | Rosette |
| 11 | Mailles perforées diametre8/10/12 |
| 12 | Filter |

| F: TAMISAGE | |
|---|---|
| 1 | Console de la machine |
| 2 | Moteur de vibration |
| 3 | maille diamètre 10 mm |
| 4 | maille diamètre 4 mm |

| G : DEPOUSSIERAGE | |
|---|---|
| 1 | moteur électrique |
| 2 | Variateur |
| 3 | Volant de régulation de la vitesse |
| 4 | Ceinture de transmission |
| 4-A | Boussole cote moteur |
| 5 | Ceinture |
| 6 | Support |
| 7 | Poulie cote arbre |
| 7-A | Boussole cote arbre |

| H : MELANGEUR | |
|---|---|
| 1 | Moteur électrique/ KW 5,5 4P 85 - 400V - 50Hz |
| 2 | Réducteur à axes parallèle |
| 3 | Joint de dépoussiérage |
| 4 | Filter |
| 5 | Boussole de traction |
| 6 | Bagues à billes |
| 7 | Bagues FRB |
| 8 | Poulie cote arbre |
| 9 | Boussole cote arbre |
| 10 | Pelle du mélangeur |
| 11 | Bras mélangeur opposes |

| I : ALIMENTATION DE LA PRESSE | |
|---|---|
| 1 | Moteur électrique KW 5,5 4P 85 - 400V- 50Hz |
| 2 | Réducteur à axes parallèles |
| 3 | Joint de dépoussiérage |
| 4 | Filter |
| 5 | Boussole de traction |
| 6 | Bagues à billes |
| 7 | Bagues FRB |
| 8 | Poulie cote arbre |
| 9 | Boussole cote arbre |
| 10 | Pelle du mélangeur |
| 11 | Bras mélanqeur opposes |

| I1 : LA PRESSE | |
|---|---|
| 1 | Moteur électrique |
| 2 | Réducteur |
| 3 | Arbre agitateur |
| 4 | Pelle agitatrice |
| 5 | Support avec bague en bronze |
| 6 | Porte de réglage |
| 7 | Palette indicatrice |
| 8 | Limiteur électromécanique |
| 9 | Moteur réducteur et variateur |

| **J** : décantation de l'huile | |
|---|---|
| | Bac de récupération et décantation en inox pompe |

| M: FILTRATION 2 | |
|---|---|
| | Plate-forme de filtration en série avant conditionnement Filtres en inox |
| | Filtres en bio polymères cellulosique |
| | Canalisation en inox |
| | Pompes |

| N:POMPAGE/SOTOKAGE | |
|---|---|
| | Pompe |
| | Tuyauteries alimentaires en bio polymères Fut de 100L |

### Les avantages de l'invention

La durée du procédé est optimale et relativement courte, 4 heures par rapport à celle des procédés des concurrents (plus de 16heures), pour une tonne de matière première.
Le procédé est continu 24/24 365j/365

La maintenance est rapide.

La qualité sanitaire organoleptique de l'huile est meilleure que celle des autres procédés: Acidité est maitrisée et acceptable 0,3 mg de potasse pour 100 g d'huile; La couleur est jaune claire.

L'huile est très homogène acceptable sans suspension ni dépôt.

L'odeur est appréciée et sans brûlure ni composés volatiles suspects.
Le rendement d'extraction est de 3,5 à 4%
Une tonne de fruits produit 48% d'amande
52% de coque
Les poussières petites et moyennes sont de 24% soit 240Kg
La masse d'huile est de 35 à 40 Kg soit un pourcentage de 4%

Le sous-produit d'amande, la coque, est riche en fibres et très pauvre en huile, est utilisée comme additif pour favoriser la pression de l'huile à partir des deux fractions broyats dans la vis sans fin.

D'après les analyses certifiées et accréditées par un laboratoire international, les résultats trouvés prouvent que la qualité initiale du fruit a été conservée et que le procédé a peu d'impact négatif sur la qualité de l'huile.

Ci-dessous les résultats des principales analyses:

| **Qualité organoleptique :** |
|---|
| La couleur est d'une couleur jaune très pale limpide |
| L'odeur ne présente aucune caractéristique ni de brulure ni de combustion ou de « cramage » |
| Lovibond rouge 7,4 0,10 |
| Lovibond bleue < LoQ 0,10 |
| Lovibond neutre < LoQ 0,10 |
| Aocs rouge 6,5 0,10 |
| Aocs jaune 70,0 0,10 |
| Lovibond jaune 70,0 0,10 |

| ACIDITE TOTALE: 0,37±0,04 g/100 g (par rapport à l'acide oleique) |
|---|
| Composition acidité titrable |
| Acide butyrique (C 4:0) n.r. % 0,010 0,050 |
| Acide capronique (C 6:0) n.r. % 0,010 0,050 |
| Acide heptanoique (C 7:0) n.r. % 0,010 0,050 |
| Acide caprilique (C 8:0) n.r. % 0,010 0,050 |
| Acide caprinique (C 10:0) n.r. % 0,010 0,050 |
| Acide caproleique (C 10:1) n.r. % 0,01O 0,050 |
| Acide laurique (C 12:0) n.r. % 0,010 0,050 |
| Acide lauroleique (C 12:1) n.r. % 0,010 0,050 |
| Acide tridecanoique (C 13:0) n.r. % 0,010 0,050 |
| Acide tridecenoique (C 13:1) n.r. % 0,010 0,050 |
| Acide miristique (C 14:0) 0,16±0,04 % 0,010 0,050 |
| Acide miristoleique (C 14:1) n.r. % 0,010 0,050 |
| Acide pentadecanoique (C 15:0) 0,07±0,04 % 0,010 0,050 |
| Acide pentadecenoique (C 15:1) n.r. % 0,010 0,050 |
| Acide palmitique (C 16:0) 12,58±0,74 % 0,010 0,050 |
| Acide palmitoleique (C 16:1) 0,09±0,04 % 0,010 0,050 |
| Acide trans esadecenoique (C16:1 trans) n.r. % 0,010 0,050 |
| Acide heptadecanoique (C 17:0) 0,15±0,04 % 0,010 0,050 |
| Acide heptadecenoique (C 17:1) 0,06±0,04 % 0,010 0,050 |
| Acide stearique (C 18:0) 5,62±0,40 % 0,01O 0,050 |
| Acide oleique (C 18:1) 44,84±1 ,28 % 0,010 0,050 |
| Acide elaidinique (C18:1 trans) tracce % 0,010 0,050 |
| Acide linoleique (C 18:2) 35,40±1 ,09 % 0,010 0,050 |
| Acide linoelaidinique (C18:2 trans) tracce % 0,010 0,050 |
| Acide linolenique (C 18:3) 0,15±0,04 % 0,010 0,050 |
| Acide trans-octadecatrienoique (C18:3 ail trans) n.r. % 0,010 0,050 |
| Acide arachique (C 20:0) 0,42±0,05 % 0,010 0,050 |
| Acide eicosenoique (C 20: 1) 0,30±0,04 % 0,010 0,050 |
| Acide beenique (C 22:0) 0,19±0,04 % 0,010 0,050 |
| Acide erucique (C 22:1) n.r. % 0,010 0,050 |
| Acide lignocerique (C 24:0) n.r. % 0,010 0,050 |
| Acide gras poly insaturé maggiori di C 20 n.r. % 0,010 0,050 |
| Acides gras saturés 19,19±0,85 % 0,010 0,050 |
| Acides gras mono insaturés 45,29±1,28 % 0,010 0,050 |
| Acides gras mono insaturés trans n.r. % 0,010 0,050 |
| Acides gras poly insaturés 35,55±1,09 % 0,010 0,050 |
| Acides gras polyinsaturés trans n.r. % 0,010 0,050 |

| Composition stérolique |
|---|
| Met.: REG CEE 2568/1991 |
| Cholestérol 0,1 JO , 0,3] % 0,10 |
| Brassicasterol < LoQ % 0,10 |
| 24-methylene cholestérol < LoQ % 0,10 |
| Campesterole 0,4±0,2 % 0,1O |

| Résultats Analytiques |
|---|
| Campestanol 0,5±0,2 % 0,10 |
| Stigmasterole 0,2±0,1 % 0,10 |
| Delta-7-campesterol 1,1±0,3 % 0,10 |
| Delta-5,23-stigmastadienole 0,8±0,3 % 0,10 |
| Cholerosterol 0,2 ] 0 , 0,4] % 0,10 |
| Beta-sitosterol < LoQ % 0,10 |
| Sitostanol 0,7±0,3 % 0,10 |
| Delta-5-avenasterol 0,2±0,1 % 0,10 |
| Delta-7,9(11)-stigmastadienol 2,9±0,5 % 0,10 Delta-5,24-stigmastadienol 1,2±0,3 % 0,10 |
| Delta-7-stigmastenol < LoQ % 0,10 |
| Delta-7-avenasterol 3,5±0,5 % 0,10 |
| Sterol total 2 008,2±302,2 mg/kg 10 |

| TOCOFEROL/ Met.: selon ISO 9936:2011 |
|---|
| Alfa-tocoferol 41±6 mg/kg 1,0 |
| Beta-tocoferol < LoQ mg/kg 1,0 |
| Delta-tocoferol 60±9 mg/kg 1,0 |
| Gamma-tocoferol 505±77 mg/kg 1,0 |
| Tocoferol total 606±78 mg/kg 1,0 |

| |
|---|
| INSAPONIFIABLE 0,60±0,09 g/100 g 0,010 |
| VITAMINA 02 n.r. µg/kg 5,010 103.7 /Met.: MP 1570 rev 2 |
| VITAMINE 03 n.r. pg/kg 5,010 98.6/ Met.: MP 1570 rev - |
| CAROTENOIDE / Met.: MP 2078 rev 2 |
| Astaxantine < LoO mg/kg 0,30 |
| Luteine < LoO mg/kg 0,30 |
| Zeaxantin < LoO mg/kg 0,30 |
| Cantaxantine < LoQ mg/kg 0,30 |
| beta-apo-8'-carotenale (E160E) < LoO mg/kg 0,30 |
| Citranaxantine < LoQ mg/kg 0,30 |
| Acide beta-apo-8'-carotenico-ethylester (E 160 f) < LoQ mg/kg 0,30 |
| beta-carotene totale 0,34±0,20 mg/kg 0,30 |
| Licopene < LoQ mg/kg 0,30 |
| Xantophyle non identifié 1,26±0,24 mg/kg 0,30 |
| Pigment total HPLC 1,60±0,31 mg/kg 0,30 |
| Pigment total SPT 1,74±0,22 mg/kg (corne beta- 0,30 carotène) |

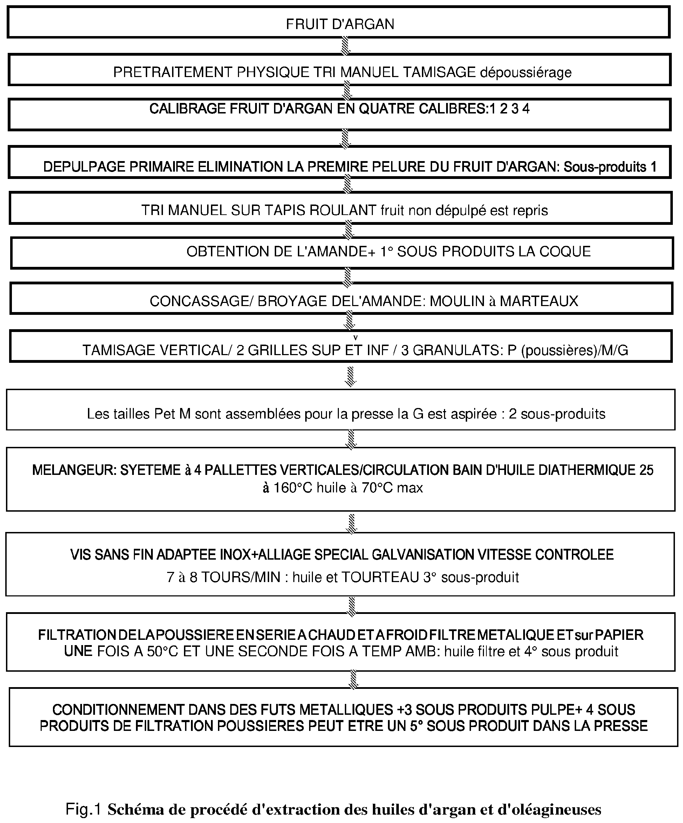

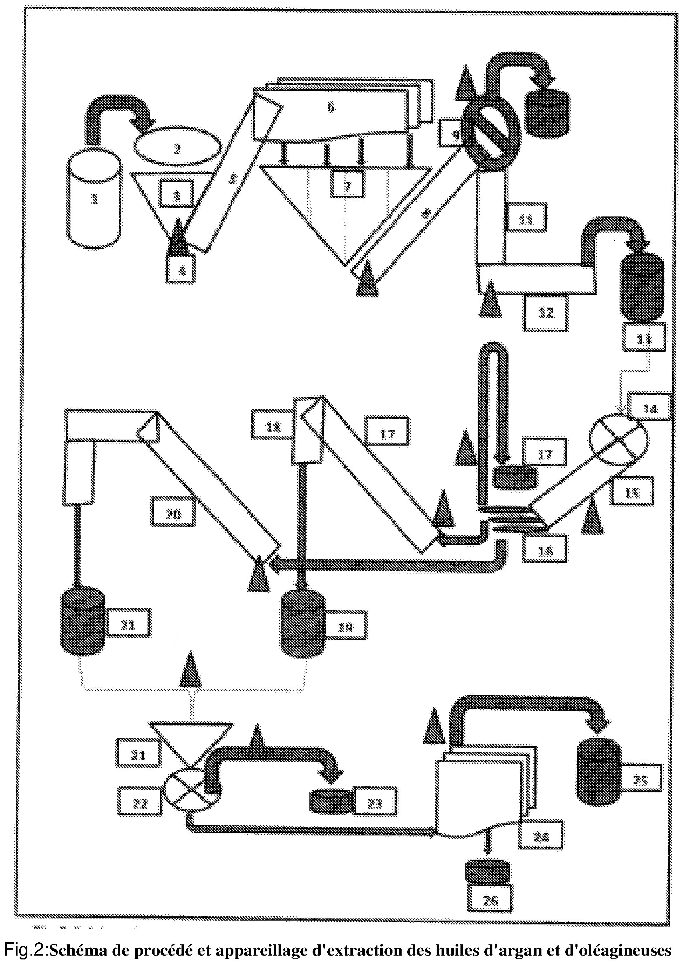

**Description de la figure 2:**

| | | | |
|---|---|---|---|
| 1: | matière première | 18: | système d'éjection |
| 2: | tamiseur | 19: | fraction fine |
| 3: | trémie | 20: | séparateur d'air |
| 4: | moteur | 21: | malaxeur |
| 5: | canal | 22: | vis sans fin |
| 6: | calibrage | 23: | contour de stockage |
| 7: | trémie | 24: | filtration à cartouche |
| 8: | canal | 25: | filtration en bio cellulose |
| 9: | dépulpage | | |
| 10: | stockage | 26: | emballage de |
| 14: | marteaux | | conservation |
| 15: | moteur | 27: | résidu solide |
| 16: | deux tamiseurs | | |
| 17: | fraction frande | | |
| 11: | motovibrateur | | |
| 12: | tapis d'entrainement | | |
| 13: | stockage des amandes | | |

## Revendications

1. Procédé d'extraction de l'huile d'argan extra vierge en continu sans rupture 24h/24 et 7jours/ 7comprenant les étapes suivantes:
a. Réception/ Tamisage: la matière première (1) est usinée tout fraichement sans aucun traitement ni déshydratation et le procédé est tributaire du taux d'humidité qui varie de 8 à 15% ; donc la vitesse, la puissance des moteurs, la fréquence, le débit, le voltage et la consommation en énergie sont réajustés en fonction de la qualité des fruits ; les fruits sont pesés afin de contrôler le taux d'extraction et le calcul des pertes ; les fruits sont triés par un contrôle visuel et manuel puis par un tamiseur (2) pour se débarrasser des poussières et des corps étrangers ;
b. Calibrage: les fruits sont placés dans une trémie (3) puis canalisés (5) grâce à un moteur pour être calibrés (6)- Quatre calibres sont obtenus ; chaque calibre est traité séparément dans la plupart des cas ;
c. Dépulpage: Le fruit est conduit vers le dépulpage (9) qui est basé sur des forces centrifuges appliquées par des palettes qui tournent horizontalement à grande vitesse ; le fruit et selon ses dimensions et son calibre est éjecté ou propulsé par une vitesse et une force proportionnelle relative sur la paroi métallique qui entraine la rupture de la coque ; la coque est aspirée par une pompe vers le stokage (10) ; l'amande est entrainée par pesanteur verticalement (11) puis subit une double vibration ;
d. Contrôle de la qualité du dépulpage: l'amande est entrainée sur un tapis (12) de contrôle visuel et manuel ; la surchauffe est contrôlée et l'amande sort tempérée et non chaude ;
e. Concassage: l'étape la plus importante est le concassage où l'amande subit un matraquage par un système de coups par marteaux (14) ;
f. Tamisage: le broyat est tamisé en 3 granulats par 2 tamiseurs (16) ; la plus fine est celle qui est la plus riche en huile (21) elle est véhiculée par un moteur ; la fraction moyenne est récupérée pour être mélangée avec la fraction la plus fine (21) ; la fraction grande est entrainée pour être séparée en deux autres fractions (17) ; la séparation est effectuée avec un système d'éjection (18) ;
g. Raffinage ou dépoussiérage: les petites fractions légères et les sous-produits ou déchets sont récupérés ; la fraction fine appelée poussières est très riche en huile (19) ; l'autre fraction correspond à des particules de grosses tailles pauvres en huile, correspond à un sous-produit qui est la coque de l'amande ou pelure ; les fractions les plus riches en huile sont transférées portées à la salle de presse ;
h. Mélangeur: les fractions (19) et (21) sont mélangées dans un malaxeur (21); il est lié à la presse pour l'alimentation par un système pneumatique de canalisation ; il est équipé d'un frein afin de ne pas créer une désynchronisation avec la presse ;
i. Alimentation de la presse: la presse est articulée avec le malaxeur de façon automatique ; elle est autorégulée pour un débit d'approvisionnement précis ; l'alimentation en matière première est sous contrôle: quantité, humidité et granulométrie ; elle donne le signal au malaxeur pour s'approvisionner en broyat ; une vis sans fin (22) tourne à une fréquence contrôlée ; des sous-produits nommés les tourteaux sont éjectés horizontalement puis propulsés d'un bac vers un conteneur de stockage (23) ;
i₁) Presse: Elle est sous contrôle physique ; la température est réglée et maitrisée selon les besoins soit cosmétique soit alimentaire, et ce par un système à 4 plateaux montés verticalement ; le contrôle des moteurs des pompes, la puissance la fréquence et l'énergie ;
i₂) Vis sans fin de la presse: la vis est en acier galvanisé et email avec un alliage spécial ;
j. Pré-filtrations: l'huile est récupérée verticalement en dessous des fentes de la vis horizontale filtrée à travers un filtre ; elle est ensuite décantée ; le surnagent est pompé par deux pompes à travers des canalisations vers la filtration (24) ; le dépôt solide est un sous-produit qui est valorisé en cosmétique ;
k. Pompage de l'huile (24): l'huile est pompée et canalisée vers la zone de filtration ;
l. Filtration 1 : l'huile subit deux séries de filtration à chaud à 40°C et à froid ;
m. Filtration 2 : la seconde à travers des filtres en bio-cellulose (25) ; un autre sous- produit résidu solide est récupéré pour la valorisation en cosmétique (27) ;
n. Pompage et stockage: l'huile est récupérée par pompage dans des futs b(26).

2. Procédé selon la revendication 1 **caractérisé en ce que** le dépulpage c) consiste à enlever la coque ou la pulpe du fruit avec une vitesse liée à la taille du fruit par des rotations horizontales des palettes qui propulsent le fruit sur la paroi métallique pour ainsi libérer l'amande ; ce dépulpage est non destructeur et basé sur la cassure et élimination de la coque superficielle sans pour autant endommager les amandes.

3. Procédé selon la revendication 2 **caractérisé en ce que** l'amande est libérée de manière descendante, et la coque est éjectée de manière ascendante ; les amandes sont ensuite véhiculées sur le tapis afin de faciliter le contrôle visuel et manuel ; un système de vibration est appliqué aux amandes lors de leur descente ; le contrôle de la qualité de dépulpage est visuel et les fruits non dépulpés seront réinjectée pour un nouveau dépulpage.

4. Procédé selon la revendication 3 **caractérisé en ce que** les amandes dépulpées sont entrainées par une vis sans fin à 45° du sol sur une hauteur de 3 m environ.

5. Procédé selon la revendication 4 **caractérisé en ce que** les amandes sont placées dans une trémie puis entrainées par une vis sans fin à 45° du sol ; les amandes sont automatiquement injectées dans un moulin à marteaux pour être moulues et broyées ; la température est contrôlée pour éviter la surchauffe des broyats ; un tamis ou filtre mécanique de 0,2 mm de maille permet de reprendre les grosses tailles pour les réinjecter dans le moulin.

6. Procède selon la revendication 5 **caractérisé en ce que** le concassage (e) des amandes se fait selon une taille maximum à ne pas dépasser et sans surchauffe : obtention de fractions à tailles et granulométries différentes ; un système de tamis binaires permet la séparation de la petite et la grande taille ; la fraction à petite granulométrie est la plus riche en huile d'argan.

7. Procédé selon la revendication 6 **caractérisé en ce que** la fraction à grande granulométrie du broyat qui est faible en huile est valorisée pour récupérer une fraction appelée poussière riche en huiles par le biais d'une séparation physique par un système de pompage et avancée par une vis sans fin d'une fraction moyenne appelée poussière et une autre plus grande évacuée verticalement en pesanteur appellée grande fraction très pauvre en huile.

8. Procédé selon la revendication 7 **caractérisé en ce que** les poussières sont rassemblées avec la première petite fraction riche en huile ; la grande fraction est valorisée autrement tout en l'utilisant comme support naturel lors de la presse de l'huile des autres fractions.

9. Procédé selon la revendication 8 **caractérisé en ce que** les fractions petites et moyennes obtenus sont transférées à la presse en passant par le mélangeur, et la troisième fraction est utilisée comme support facilitant la pression quantitative des huiles.

10. Procédé selon la revendication 9 **caractérisé en ce que** seule les fractions petite et moyenne riches en huiles sont pressées ; de préférence, les fractions avec une granulométrie précise inférieure à 0,2 mm riches en huile sont placées dans un mélangeur.

11. Installation pour l'extraction de l'huile d'argan selon le procédé des revendications 1 à 10 comprenant:
- Un poste de reception et tamisage
- Un poste de calibrage
- Un poste de dépulpage
- Un poste de contrôle
- Un concasseur
- Un poste de raffinage et dépoussiérage
- Un mélangeur
- Une presse
- Des filtres
- Un poste de pompage et stockage
**caractérisée en ce qu'**elle produit des fractions riches en huile qui sont placées dans le mélangeur ; ledit mélangeur est synchronisé avec la presse par un système hydraulique.

12. Installation selon la revendication 11 **caractérisée en ce que** le flux de la presse est tributaire du flux de la matière première du broyat des amandes contenu dans le mélangeur ; ces deux postes fonctionnent de manière synchronique afin d'assurer une presse totale des huiles.

13. Installation selon la revendication 12 **caractérisée en ce que** la presse est constituée d'une vis sans fin constituée de deux matériaux, le corps est un acier inox, et un amalgame spécial à la surface de la vis.

14. Installation selon les revendications 11 à 13 **caractérisé en ce que** les variables de la presse sont contrôlées 24h/24 et 7j/7 qualité, quantité, et température selon la destinée de l'huile et son usage culinaire ou cosmétique, le système de plateaux à quatre étages, la viscosité de l'huile de chauffage, la vitesse de la vis, la pression de presse et l'usure de l'alliage superficiel de la vis.

15. Installation selon les revendications 11 à 14 **caractérisée par** une série de filtres où passe l'huile : un pré filtre post presse et une double filtration en série une à froid à travers des filtres en inox et une seconde à travers une série de membranes de cellulose.

## Patentansprüche

1. Verfahren zur kontinuierlichen Extraktion von extra nativem Arganöl ohne Unterbrechung 24 Stunden pro Tag und 7 Tage pro Woche, bestehend aus die folgenden Schritten:
a. Annahme/Sieben: das Rohmaterial (1) ist frisch verarbeitet, ohne jegliche Behandlung oder Dehydrierung; der Prozess ist abhängig vom Feuchtigkeitsgehalt, der zwischen 8 und 15 % liegt; daher werden die Geschwindigkeit, die Motorleistung, die Frequenz, der Durchfluss, die Spannung und der Energieverbrauch je nach Qualität der Früchte neu angepasst; die Früchte werden gewogen, um den Extraktionsgrad zu kontrollieren und die Verluste zu berechnen; die Früchte werden durch Sicht- und Handkontrolle und anschließend durch eine Siebmaschine (2) sortiert, um Staub und Fremdkörper zu entfernen;
b. Kalibrierung: die Früchte werden in einen Trichter (3) gelegt und dann mithilfe eines Motors (5) kanalisiert, um sie zu kalibrieren (6) - Es werden vier verschiedene Kaliber ermittelt; jedes Kaliber wird in den meisten Fällen separat behandelt;
c. Entpulpung: die Frucht wird zum Entpulper (9) geleitet, der auf Zentrifugalkräften beruht, die von horizontal rotierenden Schaufeln mit hoher Geschwindigkeit ausgeübt werden; die Frucht wird je nach Größe und Kaliber durch Geschwindigkeit und eine proportionale Relativkraft auf die Metallwand geschleudert oder getrieben, was zum Bruch der Schale führt; die Schale wird von einer Pumpe in den Lagerraum (10) gesaugt; die Mandel wird durch die Schwerkraft vertikal mitgerissen (11) und dann einer doppelten Vibration unterzogen;
d. Qualitätskontrolle bei der Entpulpung: der Mandelkern wird auf ein Band (12) zur visuellen und manuellen Kontrolle befördert; die Überhitzung wird kontrolliert und die Mandel kommt temperiert und nicht heiß heraus;
e. Zerkleinerung: der wichtigste Schritt ist die Zerkleinerung, bei der die Mandel durch ein Hammerschlagsystem zerkleinert wird (14);
f. Sieben: das Mahlgut wird von zwei Siebmaschinen (16) in 3 verschiedene Granulate gesiebt; die feinste ist die mit dem höchsten Ölgehalt (21); die wird von einem Motor befördert; die mittlere Fraktion wird gesammelt, um mit der feinsten Fraktion (21) vermischt zu werden; die große Fraktion wird angetrieben, um in zwei weitere Fraktionen getrennt zu werden (17); die Trennung wird mit einem Auswurfsystem (18) durchgeführt;
g. Raffination oder Entstaubung: Kleine, leichte Fraktionen und Nebenprodukte oder Abfälle werden aufgefangen; die feine Fraktion, die als Staub bezeichnet wird, ist sehr reich an Öl (19); die andere Fraktion besteht aus großen, ölarmen Partikeln und entspricht einem Nebenprodukt, der Mandelschale oder Schale; die ölreichsten Fraktionen werden in den Pressraum überführt;
h. Mischapparat: die Fraktionen (19) und (21) werden in einem Mischer (21) gemischt; er ist über ein pneumatisches Rohrleitungssystem mit der Beschickungspresse verbunden; er ist mit einer Bremse ausgestattet, damit es nicht zu einer Desynchronisation mit der Presse kommt;
i. Beschickung der Presse: die Presse ist automatisch mit dem Mischer verbunden; sie ist selbstregulierend und sorgt so für eine präzise Versorgungsrate; die Rohstoffzufuhr ist unter Kontrolle: Menge, Feuchtigkeit und Korngröße; sie gibt dem Mischer das Signal, sich mit Mahlgut zu versorgen; eine Schnecke (22) dreht sich mit kontrollierter Frequenz; nebenprodukte wie Presskuchen werden horizontal ausgeworfen und dann von einem Behälter in einen Lagerbehälter (23) befördert;
i₁) Presse: Sie steht unter physischer Kontrolle; die Temperatur wird je nach Bedarf entweder für Kosmetika oder Lebensmittel reguliert und kontrolliert, und zwar durch ein System mit vier vertikal montierten Platten; die Steuerung der Pumpenmotoren, die Leistung, die Frequenz und die Energie;
i₂) Pressenschnecke: Die Schnecke besteht aus verzinktem Stahl und ist mit einer speziellen Legierung emailliert;
j. Vorfiltration: das Öl wird vertikal unter den Schlitzen der horizontalen Schnecke aufgefangen und durch einen Filter gefiltert; Anschließend wird sie dekantiert; der Überstand wird von zwei Pumpen durch Leitungen zur Filtration (24) gepumpt; die feste Ablagerung ist ein Nebenprodukt, das in der Kosmetik verwertet wird;
k.Abpumpen des Öls (24): das Öl wird abgepumpt und in den Filterbereich geleitet;
l. Filtration 1: das Öl durchläuft zwei Serien von Heißfiltration bei 40°C und einer Kaltfiltration;
m. Filtration 2: die zweite durch Biozellulosefilter (25); ein weiteres Nebenprodukt, ein fester Rückstand, wird für die Verwertung in Kosmetika gewonnen (27);
n. Abpumpen und Lagerung: das Öl wird in B-Fässer (26) gepumpt.

2. Verfahren nach Anspruch 1, **dadurch gekennzeichnet, dass** das Entpulpen c) darin besteht, die Schale oder das Fruchtfleisch der Frucht mit einer Geschwindigkeit zu entfernen, die mit der Größe der Frucht zusammenhängt, und zwar durch horizontale Drehungen der Paletten, die die Frucht gegen die Metallwand treiben, um dadurch den Kern freizusetzen; dieses Entpulpen ist schonend und basiert auf dem Aufbrechen und Entfernen der oberflächlichen Schale, ohne die Mandeln zu beschädigen.

3. Verfahren nach Anspruch 2, **dadurch gekennzeichnet, dass** die Mandel nach unten freigegeben wird und die Schale nach oben ausgeworfen wird; die Mandeln werden dann auf das Band befördert, um die visuelle und manuelle Kontrolle zu erleichtern; beim Absenken der Mandeln wird ein Vibrationssystem angewendet; die Kontrolle der Entpulpungsqualität ist visuell und die nicht entpulpten Früchte werden für eine erneute Entpulpung wieder eingezogen.

4. Verfahren nach Anspruch 3, **dadurch gekennzeichnet, dass** die entpulpten Mandeln von einer Schnecke in einem Winkel von 45° zum Boden auf eine Höhe von etwa 3 m gebracht werden.

5. Verfahren nach Anspruch 4, **dadurch gekennzeichnet, dass** die Mandeln in einen Trichter gegeben und dann von einer Schnecke in einem Winkel von 45° zum Boden angetrieben werden; die Mandeln werden automatisch in eine Hammermühle eingeleitet, wo sie gemahlen und zerkleinert werden; die Temperatur wird kontrolliert, um eine Überhitzung des Mahlguts zu vermeiden; ein mechanisches Sieb bzw; filter mit einer Maschenweite von 0,2 mm nimmt die großen Stücke auf, um sie erneut in die Mühle einzuleiten.

6. Verfahren nach Anspruch 5, **dadurch gekennzeichnet, dass** das Zerkleinern (e) der Mandeln gemäß einer maximalen Größe, die nicht überschritten werden darf, und ohne Überhitzung erfolgt; gewinnung von Fraktionen mit unterschiedlichen Maßen und Korngrößen; ein binäres Siebsystem ermöglicht die Trennung von kleinen und großen Größen; die Fraktion mit kleiner Korngröße ist am reichsten an Arganöl.

7. Verfahren nach Anspruch 6, **dadurch gekennzeichnet, dass** die großkörnige Fraktion des Mahlguts, die wenig Öl enthält, zur Gewinnung einer Fraktion, die als ölreicher Staub bezeichnet wird, durch physikalische Trennung mittels eines Pumpsystems und Förderung durch eine Schnecke einer mittleren Fraktion, die als Staub bezeichnet wird, und einer anderen größeren Fraktion, die vertikal unter Schwerkraft abgeführt wird und als große, sehr ölarme Fraktion bezeichnet wird, verwertet wird.

8. Verfahren nach Anspruch 7, **dadurch gekennzeichnet, dass** der Staub mit der ersten kleinen ölreichen Fraktion zusammengeführt wird; die große Fraktion wird auf andere Weise verwertet, während sie gleichzeitig als natürlicher Träger beim Pressen des Öls aus den anderen Fraktionen verwendet wird.

9. Verfahren nach Anspruch 8, **dadurch gekennzeichnet, dass** die erhaltenen kleinen und mittleren Fraktionen über den Mischer in die Presse überführt werden und die dritte Fraktion als Trägermaterial verwendet wird, das das quantitative Auspressen der Öle erleichtert.

10. Verfahren nach Anspruch 9, **dadurch gekennzeichnet, dass** nur die kleinen und mittleren ölreichen Fraktionen gepresst werden; vorzugsweise werden die ölreichen Fraktionen mit einer genauen Korngröße von weniger als 0,2 mm in einen Mischer gegeben.

11. Vorrichtung zur Gewinnung von Arganöl nach dem Verfahren der Ansprüche 1 bis 10, die Folgendes umfasst:
- Eine Annahme- und Siebstation
- Eine Kalibrierstation
- Eine Entpulpungsstation
- Eine Kontrollstation
- Ein Brecher
- Eine Raffinier- und Entstaubungsstation
- Ein Mischer
- Eine Presse
- Filter
- Eine Pump- und Lagerstation
sie ist **dadurch gekennzeichnet, dass** sie ölreiche Fraktionen produziert, die in den Mischer gegeben werden; der besagte Mischer wird über ein Hydrauliksystem mit der Presse synchronisiert.

12. Vorrichtung nach Anspruch 11, **dadurch gekennzeichnet, dass** der Durchfluss der Presse vom Durchfluss des im Mischer enthaltenen Mandelmahlguts abhängig ist; diese beiden Stationen arbeiten synchron, um eine vollständige Auspressung der Öle zu gewährleisten.

13. Vorrichtung nach Anspruch 12, **dadurch gekennzeichnet, dass** die Presse aus einer Schnecke aufgebaut ist, die aus zwei Materialien besteht: der Körper aus Edelstahl und einem speziellen Amalgam auf der Oberfläche der Schnecke.

14. Anlage nach den Ansprüchen 11 bis 13, **dadurch gekennzeichnet, dass** die Pressenvariablen rund um die Uhr auf Qualität, Quantität und Temperatur je nach Bestimmung des Öls und seiner kulinarischen oder kosmetischen Verwendung, dem vierstufigen Tablettsystem, der Viskosität des Heizöls, der Schneckengeschwindigkeit, dem Pressdruck und dem Verschleiß der Oberflächenlegierung der Schnecke kontrolliert werden.

15. Anlage nach den Ansprüchen 11 bis 14, **gekennzeichnet durch** eine Reihe von Filtern, durch die das Öl strömt; ein Vorfilter nach der Presse und eine doppelte Serienfiltration, eine in kaltem Zustand durch Edelstahlfilter und eine zweite durch eine Reihe von Zellulosemembranen.

## Claims

1. The process for extracting extra virgin argan oil continuously without interruption 24/7, which consists of the following steps:
a. reception/ sifting: the raw material (1) is processed freshly without any treatment or dehydration, and the process is dependent on the moisture content, which varies from 8 to 15%; so the speed, motor power, frequency, flow, voltage and energy consumption are readjusted according to the quality of the fruits; the fruits are weighed to control the extraction rate and to calculate the waste; the fruits are sorted by visual and manual inspection, and then go through a sifter (2) to remove any dust and foreign matter;
b. grading: the fruit is placed in a hopper (3), then channeled (5) by a motor to be graded (6) - four grades are obtained; each grade is processed separately in most cases;
c.pulping: the fruit is led to the pulping unit (9), which is based on centrifugal forces applied by paddles rotating horizontally at high speed; depending on its size, the fruit is ejected or propelled by a speed and a relative proportional force against the metal wall, which causes the shell to break; the shell is sucked up by a pump into a storage area (10); the kernel is driven vertically by gravity (11) and then undergoes a double vibration;
d. pulping quality control: the kernel is driven onto a conveyor belt (12) for visual and manual control; the overheating is controlled and the kernel comes out temperate and not hot;
e. crushing: the most important stage is crushing, where the kernels are crushed by a hammer system (14)
f. sifting: the crushed material is sifted into 3 granules by 2 sifters (16); the finest is the one that is richest in oil (21), it is transported by an engine; the medium fraction is recovered and mixed with the finest fraction (21); the large fraction is driven to be separated into two other fractions (17); the separation is carried out with an ejection system (18);
g. refining or dusting: small light fractions and by-products or waste are recovered; the fine fraction called dust is very rich in oil (19); the other fraction, made up of large particles with a low oil content, is a by-product known as the almond shell or skin; the fractions richest in oil are transferred to the press room;
h. mixer: fractions (19) and (21) are mixed in a mixer (21); it is connected to the press for feeding by a pneumatic pipeline system; it is equipped with a brake so as not to create desynchronization with the press;
i. press feed: the press is automatically linked to the mixer; it is self-regulating for a precise supply rate; the supply of raw material is under control: quantity, humidity and particle size; it gives the signal to the mixer to supply the crushed material; an endless screw (22) rotates at a controlled frequency; by-products called cakes are ejected horizontally and then propelled from a bin to a storage container (23);
i₁) press: it is under physical control; the temperature is adjusted and controlled according to the cosmetic or feed requirements, by a system with 4 vertically mounted trays; pump motor control, power frequency and energy;
i₂) endless screw on the press: the screw is made of galvanized steel and enamel with a special alloy;
j. pre-filtrations: the oil is recovered vertically beneath the horizontal screw slots and filtered through a filter; it is then decanted; the supernatant is pumped by two pumps through pipelines to the filtration (24); the solid deposit is a by-product which is used in cosmetics;
k.oil pumping (24): the oil is pumped and channeled to the filtration zone
l. filtration 1: the oil undergoes two series of filtration: hot filtration at 40°c and cold filtration;
m. filtration 2: the second one goes through bio-cellulose filters (25); another by-product, solid residue, is recovered for use in cosmetics (27);
n. pumping and storage: the oil is recovered by pumping it into drums b(26).

2. The process according to claim 1, is **characterized by** pulping c) which consists of removing the shell or the pulp of the fruit at a speed related to the size of the fruit by horizontal rotations of the paddles, which propel the fruit onto the metal wall, thereby releasing the kernel; the pulping process is non-destructive and based on breaking and removing the surface shell without damaging the kernels.

3. The process according to claim 2, is **characterized by** the kernel being released downwards, and the shell being ejected upwards; the kernels are then transported onto the conveyor belt to facilitate visual and manual inspection; a vibration system is applied to the kernels as they descend; the pulping quality is checked visually, and any fruit that has not been pulped is re-injected to be pulped again.

4. The process according to claim 3, is **characterized by** the pulped kernels being driven by an endless screw at a 45° angle to the ground at a height of approximately 3 m.

5. The process according to claim 4, is **characterized in that** the kernels are placed in a hopper and then driven by an endless screw at a 45° angle to the ground; the kernels are automatically injected into a hammer mill to be ground and crushed; the temperature is controlled to avoid overheating of the crushed material; a 0;2 mm mesh sieve or mechanical filter collects the larger sizes and re-injects them back into the mill.

6. The process according to claim 5, is **characterized in that** the kernels are crushed (e) according to a maximum size which cannot be exceeded and without overheating them; fractions of different sizes and particle sizes are obtained; a binary sieve system is used to separate the small and large sizes; the smaller particle size fraction is the richest in argan oil.

7. The process according to claim 6, is **characterized in that** the large particle size fraction of the ground material, which is low in oil, is used to recover a fraction called oil-rich dust by means of physical separation, by a pumping system and advanced by an endless screw with a medium fraction called dust, and another larger fraction discharged vertically under gravity, called a large fraction very low in oil.

8. The process according to claim 7, is **characterized in that** the dust is collected with the first oil-rich small fraction; the large fraction is recovered in a different way, using it as a natural support when pressing the oil from the other fractions.

9. The process according to claim 8, is **characterized in that** the small and medium fractions obtained are transferred to the press via the mixer, and the third fraction is used as a support facilitating the quantitative pressing of the oils.

10. The process according to claim 9, is **characterized in that** only the small and medium fractions rich in oil are pressed; the fractions with the exact particle size of less than 0;2 mm rich in oil are preferably placed in a mixer.

11. Installation for the extraction of argan oil according to the process of claims 1 to 10, which consists of:
- a reception and sifting station
- a grading station
- a pulping station
- a control station
- a crusher
- a refining and dusting station
- a mixer
- a press
- filters
- a pumping and storage station
**characterized in that** it produces oil-rich fractions which are placed in the mixer; this mixer is synchronized with the press by a hydraulic system.

12. The installation according to claim 11, is **characterized in that** the flow of the press is dependent on the flow of the raw material of crushed almonds contained in the mixer; these two stations operate in sync to ensure that the oils are pressed completely.

13. The installation according to claim 12, is **characterized in that** the press consists of an endless screw made of two materials, the body is stainless steel, and it has a special amalgam on the surface of the screw.

14. The installation according to claims 11 to 13, is **characterized in that** the variables of the press are controlled 24/7 - quality, quantity and temperature - depending on the purpose of the oil and its culinary or cosmetic use, the four-stage tray system, the viscosity of the heating oil, the speed of the screw, the pressure of the press and the wear of the surface alloy of the screw.

15. The installation according to claims 11 to 14, is **characterized by** a series of filters through which the oil passes; a post press pre-filter and a series of double filtration, one cold through stainless steel filters and a second through a series of cellulose membranes.
